Europäisches Patentamt

European Patent Office

Office européen des brevets

(11) Publication number: **0 311 787 B1**

# EUROPEAN PATENT SPECIFICATION

(45) Date of publication of patent specification: **10.06.92**    (51) Int. Cl.⁵: **A61J 1/00**

(21) Application number: **88114726.8**

(22) Date of filing: **09.09.88**

(54) **Apparatus with safety locking members, for connecting a syringe to a bottle containing a medicament.**

(30) Priority: **14.10.87 IT 2228387**

(43) Date of publication of application:
**19.04.89 Bulletin 89/16**

(45) Publication of the grant of the patent:
**10.06.92 Bulletin 92/24**

(84) Designated Contracting States:
**AT BE CH DE ES FR GB GR IT LI LU NL SE**

(56) References cited:
**CH-A- 581 273**
**FR-A- 2 560 049**

(73) Proprietor: **FARMITALIA CARLO ERBA S.r.L.
Via Carlo Imbonati 24
I-20159 Milano(IT)**

(72) Inventor: **Coccia, Mario
Via Gramsci, 25
I-20090 Boscone Milan(IT)**

(74) Representative: **Giambrocono, Alfonso, Dr.
Ing. et al
Ing. A. Giambrocono & C. S.r.l. Via Rosolino
Pilo 19/B
I-20129 Milano(IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

The invention relates to an improvement in apparatus for connecting a bottle containing a medicament to a syringe used to withdraw the medicament from the bottle, possibly after feeding into this latter a solvent or the like.

More specifically, the said apparatus is of the type described in USA patent No. 4,576,211. It comprises several mutually mobile constituent parts and at one end contains a seat into which the end of a syringe can be fitted so that its needle extends into a closed chamber bounded by the apparatus which, at its other end, comprises a seat for housing the mouth of a bottle containing a medicament. The various constituent parts of the apparatus can move axially along each other to securely lock the bottle mouth in its seat by means of retention teeth projecting from flexible tongues, and to cause the point and a portion of the needle to emerge from its chamber so that the needle can perforate the elastic plug fitted to the bottle opening and penetrate into the bottle, to be able to inject into it any liquid contained in the syringe or to draw into the syringe the liquid contained in the bottle.

While the bottle is connected to the apparatus the needle is always contained within the apparatus chamber or (partly) within the bottle, ie the needle is never freely exposed and accessible from the outside.

The apparatus of USA patent 4,576,211 has however the drawback that if no bottle is inserted into its seat in the apparatus, this can still be erroneously or accidentally operated in the sense of moving the various component parts along each other to cause the needle to emerge from its protection chamber. In such a case the needle would project outwards from the apparatus and be accessible from the outside, with consequent danger in terms of loss of needle sterility and even greater danger if a high-risk medicament such as an antitumoral drug has already been drawn into the syringe.

The object of the present invention is therefore to obviate the aforesaid drawbacks, ie to prevent the various constituent parts of the apparatus being able to move along each other when no bottle mouth (or the like) is inserted into its seat in the apparatus, to thus prevent the needle emerging from the closed chamber provided in the apparatus.

This is attained by an apparatus comprising several constituent parts and having at one end a first seat for housing the end of a syringe the needle of which extends into a closed chamber in said apparatus, at the other end of which there is provided a second seat for housing the mouth of a bottle or the like, said constituent parts being mobile along each other between a travel stop at which the needle is entirely housed within said chamber and a travel stop at which the free end of the needle and a portion thereof emerge from said chamber to extend through a perforable plug and into said second seat, the apparatus being characterised in that from one of said constituent parts there extends a flexible appendix comprising at least one protuberance projecting into said second seat, another of said constituent parts being provided with a stop tooth positioned in front, and in the immediate vicinity, of the free end of said flexible appendix when in its rest position, in which the appendix abuts against said stop tooth and does allow movement of said constituent parts along each other only to a point at which the needle does not emerge from the plug, whereas when a bottle is inserted into said second seat, such bottle acts against said protuberance radially moving outwardly the appendix to a position which allows further movement of the constituent parts along each other so that the needle can extend through the plug.

The structure and characteristics of the apparatus will be more apparent from the description given hereinafter by way of nonlimiting example with reference to the accompanying drawing, in which:

Figure 1 is a partly sectional view of the apparatus in its rest state, with the needle completely retracted into the closed chamber of the apparatus;

Figure 2 shows the same apparatus connected to a bottle mouth and with the syringe needle projecting from the closed chamber and extending into the bottle; and

Figure 3 shows only an end portion of the apparatus in the state shown in Figure 2, but with a bottle neck of different thickness from that shown in Figure 2.

The apparatus shown on the drawing comprises three separate main constituent parts, namely the parts 1, 2 and 3. Pins 4 are rigid with one of these parts and extend radially into axially extending grooves formed in the other two parts. In this manner the three parts 1, 2 and 3 are coupled to each other but can be moved axially along each other.

The part 1 comprises a seat (not shown) to house and retain the free end of a syringe 5. With the said part 1 there is rigid a needle 6 which when the apparatus is in use is securely connected to the syringe inlet and outlet hole.

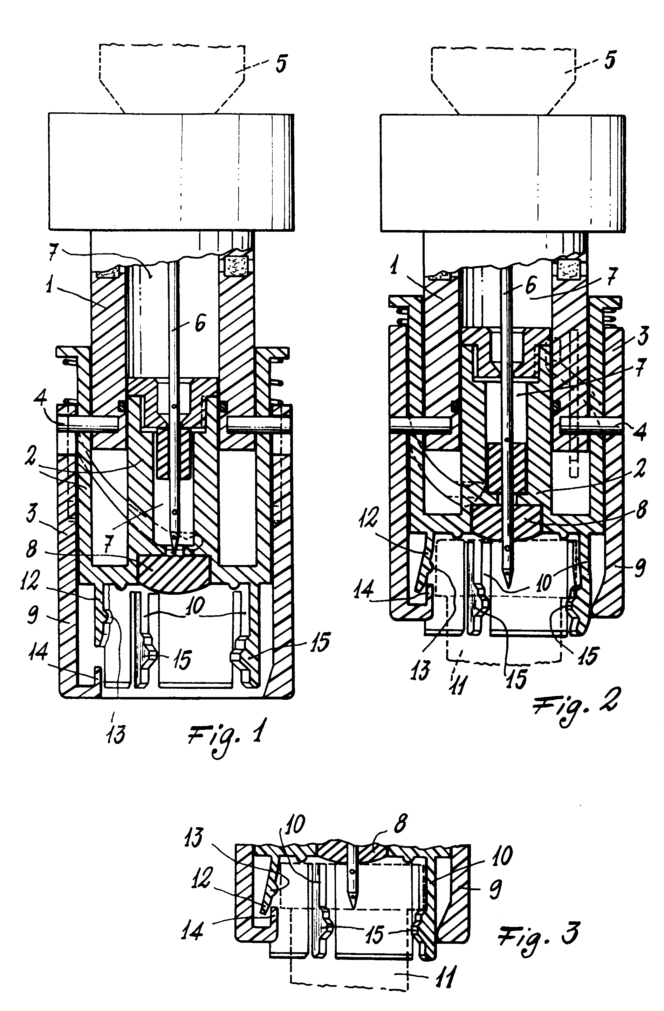

When the apparatus is in its rest state, ie not in use (Figure 1), the needle 6 is completely enclosed and protected within a chamber 7 defined by the parts 1 and 2. At its lower end, below the needle point, it comprises a hole hermetically sealed by a perforable plug 8 of rubber or a similar material.

The part 3 extends downwards from the apparatus (with reference to the figures of the drawing) beyond the part 2 in the form of a shaped continuous tubular wall 9 which has its free end enlarged in an inward direction and into which from the lower end of the part 2 there extend the flexible retention elements 10, the outer surface of which is such as not to interfere with the free edge of the wall 9 when the parts 1, 2 and 3 are superposed on each other to their maximum extent (Figure 2), provided that said appendices are free, ie provided no bottle is connected to the described apparatus.

In this respect, the retention elements 10 define a housing seat in which the mouth of a bottle 11 for containing medicaments can be inserted and retained. In Figure 1 no bottle is inserted in its housing seat, the apparatus is in its rest state and the elements 10 can bend both inwards and outwards. Under these conditions (Figure 1), if the mouth of a bottle 11 is inserted into the seat defined by the elements 10 these are bent freely outwards by the bottle neck, which interferes with the protuberances projecting from these elements. When the bottle 11 has been inserted into its seat in the described manner, the parts 1, 2 and 3 can be moved along each other (Figure 2) so that the free edge of the wall 9 rests against the outer surface of the elements 10, which can therefore not bend further outwards. The result is that when in this position the protuberances projecting inwards from the elements 10 are locked under the neck of the bottle, which can therefore not be removed from the apparatus. It will be noted that when the apparatus is in the position shown in Figure 1, the needle is completely housed within the chamber 7, whereas when in the position shown in Figure 2 the needle has passed through the plug and extends into the bottle 11.

The apparatus described up to this point is of well known type, and is illustrated in USA patent No. 4,576,211. This apparatus has considerable merit, but has the drawback that if no bottle is inserted between the elements 10, the parts 1, 2 and 3 can still be moved freely along each other to cause the needle to emerge from the closed chamber of the apparatus. The point of the needle and a portion thereof are therefore exposed and freely extending outside the apparatus, a situation which can be very dangerous if the syringe is filled with a potentially toxic medicament, such as an antitumoral drug.

To prevent this happening, ie to allow the needle point to pass beyond the plug 8 only when the mouth of a bottle 11 is inserted between the retention elements 10, according to the present invention there projects from the lower end of the part 2 at least one flexible appendix 12 having a protuberance 13 projecting towards the seat defined by the inner surfaces of the retention elements 10. The free end of a tooth 14 which projects upwards from the edge of the wall 9 of the part 3 is positioned in front of the free end of the appendix 12 (when in the rest state of Figure 1).

Assuming that the conditions of Figure 1 apply, if an attempt is made to slide the parts 1, 2, 3 along each other, the free end of the appendix 12 makes contact with the free end of the tooth 14 to prevent any further sliding of the parts along each other, and thus prevent the needle 6 emerging from the plug 8.

It will again be assumed that the rest conditions of Figure 1 apply, but that the mouth of a bottle 11 is now inserted into the seat defined by the flexible elements 10. As the bottle mouth is inserted into said seat, the mouth firstly acts against the protuberances 15 projecting from the elements 10 (so urging these latter outwards), and then acts against the protuberance 13, so bending the appendix 12 outwards. Under these conditions, the parts 1, 2, 3 can be freely slid along each other (and the needle can perforate and project below the plug 8) because the appendix 12 is in an outwardly bent state and its free end no longer comes into contact with the stop tooth 14. Thus the presence of the appendix 12 with its protuberance 13 and the presence and positioning of the stop tooth 14 ensure that the needle 6 can be made to project below the plug 8 (and thus be freely extending and accessible from the outside) only when a medicament bottle mouth or an object reproducing the shape of such a bottle mouth is housed between the flexible elements 10.

It should be noted that when in the state shown in Figure 2, the protuberances 15 are positioned below the lower edge (relative to the figures) of the mouth of the bottle 11, so preventing its removal from the apparatus as the elements 10 now rest against the free edge of the wall 9 and cannot therefore bend outwards.

The present invention also incorporates the following further characteristic. As stated, when in the state shown in Figure 2, the protuberances 15 hook onto and securely retain a bottle mouth.

Most bottles have a mouth with a projecting neck of constant defined height or thickness. There are however bottles with necks of a different height, for example greater that that of the bottle shown by dashed lines in Figure 2.

In the apparatus described in USA patent No. 4,576,211 the flexible elements 10 are provided with protuberances able to hook only onto bottles having necks of a defined constant height.

As can be seen from the drawings accompanying this description, the protuberances 15 are shaped stepwise with the tallest or most projecting step positioned at the free end of the elements 10. In this manner, when the bottle neck is of smaller height, it is hooked by a shorter step on the protuberances 15 (Figure 2), whereas if the bottle neck is of greater height (Figure 3) it is hooked by a taller step on said protuberances.

The apparatus is therefore more versatile than the known type and can be used with bottles of different structure.

## Claims

1. An apparatus with safety locking members, for connecting a syringe (5) to a bottle (11) containing a medicament, said apparatus comprising several constituent parts (1,2,3) and having at one end a first seat for housing the end of a syringe (5) the needle (6) of which extends into a closed chamber (7) in said apparatus, at the other end of which there is provided a second seat for housing the mouth of a bottle (11) or the like, said constituent parts (1,2,3) being mobile along each other between a travel stop at which the needle (6) is entirely housed within said chamber (7) and a travel stop at which the free end of the needle (6) and a portion there of emerge from said chamber (7) to extend through a perforable plug (8) and into said second seat, the apparatus being characterised in that from one (2) of said constituent parts (1,2,3) there extends a flexible appendix (12) comprising at least one protuberance (13) projecting into said second seat, another (3) of said constituent parts (1,2,3) being provided with a stop tooth (14) positioned in front, and in the immediate vicinity, of the free end of said flexible appendix (12) when in its rest position, in which the appendix (12) abuts against said stop tooth (14) and does allow movement of said constituent parts (1,2,3) along each other only to a point at which the needle (6) does not emerge from the plug (8), whereas when a bottle (11) is inserted into said second seat, such bottle (11) acts against said protuberance (13) radially moving outwardly the appendix (12) to a position which allows further movement of the constituent parts (1,2,3) along each other so that the needle (6) can extend through the plug (8).

2. An apparatus as claimed in claim 1, in which said second seat for housing the mouth of a bottle (11) is at least partly defined by flexible elements (10) projecting from one (2) of said constituent parts (1,2,3) towards the opening of said seat, and in which from another (3) of said parts (1,2,3) there projects a tubular wall (9) the free edge of which is substantially aligned with the outer surface of said flexible elements (10), characterised in that from each of said flexible elements (10) there inwardly projects a step-shaped protuberance (15), the step of greatest height or thickness being closer to the end of the relative flexible element (10) than the other steps.

## Revendications

1. Appareil comportant des éléments de blocage de sécurité pour relier une seringue (5) à un flacon (11) contenant un médicament, ledit appareil comprenant plusieurs éléments constituants (1, 2, 3) et présentant à une extrémité un premier siège recevant l'extrémité d'une seringue (5) dont l'aiguille (6) s'étend dans une chambre fermée (7) dudit appareil, à l'autre extrémité duquel est prévu un deuxième siège recevant l'ouverture d'un flacon (11) ou analogue, lesdits éléments constituants (1, 2, 3) étant mobiles les uns le long des autres entre une butée où l'aiguille (6) est entièrement logée dans ladite chambre (7) et une butée où l'extrémité libre de l'aiguille (6) et une partie de cette dernière sortent de la chambre (7) en traversant un bouchon perforable (8) et s'étendent dans le deuxième siège, caractérisé par le fait qu'à partir de l'un des éléments constituants (1, 2, 3) s'étend un appendice flexible (12), comprenant au moins une protubérance (13) qui s'étend dans ledit siège, un autre (3) des éléments constituants (1, 2, 3) présentant une dent de butée (14) placée en face et au voisinage immédiat de l'extrémité libre dudit appendice flexible (12) lorsque celui-ci se trouve dans la position de repos où l'appendice (12) bute contre ladite dent d'arrêt (14) et permet un déplacement des éléments constituants (1, 2, 3) les uns le long des autres seulement dans la mesure où celui-ci ne fait pas sortir l'aiguille (6) du bouchon (8), tandis que, lorsqu'un flacon (11) est introduit dans ledit deuxième siège, ce flacon agit sur la protubérance (13) en déplaçant l'appendice (12) radialement vers l'extérieur, vers une position qui permet un déplacement supplémentaire des éléments constituants (1, 2, 3) les uns le long des autres, l'aiguille (6) pouvant ainsi traverser le bouchon (8).

2. Appareil selon la revendication 1, dans lequel le deuxième siège qui reçoit l'ouverture d'un flacon (11) est au moins partiellement défini par des éléments flexibles (10) faisant saillie de l'un (2) des éléments constituants (1, 2, 3) vers l'ouverture dudit siège et dans lequel, à partir d'un autre (3) desdits éléments (1, 2, 3), fait saillie une paroi tubulaire (9), dont le bord libre est sensiblement situé au droit de la surface extérieure desdits éléments flexibles (10),

caractérisé par le fait qu'à partir de chacun desdits éléments flexibles (10) une protubérance (15) en escalier fait saillie vers l'intérieur, la marche la plus haute ou la plus épaisse étant située plus près que les autres de l'extrémité de l'élément flexible (10) correspondant.

**Patentansprüche**

1. Vorrichtung mit Sicherheitsfixierungsteilen für die Verbindung einer Spritze (5) mit einer Flasche (11) mit einem Medikament, wobei die Vorrichtung mehrere Bestandteile (1, 2, 3) umfaßt und am einen Ende einen ersten Sitz zur Aufnahme des Endes einer Spritze (5) aufweist, deren Nadel (6) sich in eine geschlossene Kammer (7) in der Vorrichtung hineinerstreckt, an deren anderem Ende ein zweiter Sitz zur Aufnahme der Mündung einer Flasche (11) oder dergleichen vorgesehen ist, wobei die genannten Bestandteile (1, 2, 3) aneinander zwischen einer Bewegungs-Endlage, an der die Nadel (6) vollständig innerhalb der genannten Kammer (7) aufgenommen ist, und einer Bewegungs-Endlage, an der das freie Ende der Nadel (6) und ein Teil von dieser aus der genannten Kammer (7) austreten, um sich durch einen perforierbaren Stopfen (8) hindurch und in den genannten zweiten Sitz zu erstrecken, entlang beweglich sind, und wobei die Vorrichtung dadurch **gekennzeichnet** ist, daß vom einen (2) der genannten Bestandteile (1, 2, 3) sich ein flexibler Ansatz (12) erstreckt, der mindestens einen Höcker (13) aufweist, der sich in den zweiten Sitz hineinerstreckt, und ein anderes (3) der genannten Bestandteile (1, 2, 3) mit einem Anschlagzahn (14) versehen ist, der vor dem freien Ende des flexiblen Ansatzes (12) und in dessen unmittelbarer Nähe angeordnet ist, wenn er sich in seiner Ruhelage befindet, wobei der Ansatz (12) gegen den Anschlagzahn (14) anschlägt und die Bewegung der genannten Bestandteile (1, 2, 3) aneinander entlang nur bis zu einem Punkt gestattet, an dem die Nadel (6) noch nicht aus dem Stopfen (8) austritt, während dann, wenn eine Flasche (11) in den genannten zweiten Sitz eingeführt ist, eine solche Flasche (11) gegen den Höcker (13) einwirkt, der dann den Ansatz (12) radial nach außen bis in eine Lage bewegt, die die noch weitere Bewegung der Bestandteile (1, 2, 3) aneinander entlang erlaubt, so daß sich die Nadel (6) durch den Stopfen (8) hindurch erstrecken kann.

2. Vorrichtung nach Anspruch 1, worin der genannte zweite Sitz zur Aufnahme der Mündung einer Flasche (11) mindestens teilweise von einem fleixblen Element (10) gebildet ist, das vom einen (2) der genannten Bestandteile (1, 2, 3) zur Öffnung des genannten Sitzes hin vorspringt, und worin von einem anderen (3) der genannten Bestandteile (1, 2, 3) aus eine Rohrwand (9) vorspringt, deren freie Kante im wesentlichen auf die äußere Oberfläche der flexiblen Elemente (10) ausgerichtet ist, dadurch **gekennzeichnet,** daß von jedem flexiblen Element (10) aus ein stufenförmiger Hökker (15) nach innen vorsteht, wobei die Stufe mit der größten Höhe oder Dicke näher am Ende des relativen flexiblen Elements (10) als die anderen Stufen liegt.

Fig. 1

Fig. 2

Fig. 3